# EUROPEAN PATENT SPECIFICATION

(11) **EP 1 429 770 B1**
(45) Date of publication and mention of the grant of the patent: **13.05.2009**
(21) Application number: 02799531.5
(22) Date of filing: 10.09.2002
(51) Int. Cl.: A61K 31/465, A61K 9/20, A61K 9/48, A61P 25/34, A61P 25/28, A61P 25/16, A61P 1/04, A61P 3/04

(54) **NEW FORMULATIONS AND USE THEREOF**
NEUE FORMULIERUNGEN UND IHRE VERWENDUNG
NOUVELLES FORMULATIONS ET LEUR UTILISATION

(30) Priority: 27.09.2001 SE 0103210
(43) Date of publication of application: 23.06.2004
(73) Proprietor: McNeil AB, 254 42 Helsingborg (SE)
(72) Inventor: LANDH, Tomas, S-226 49 Lund (SE); LINDBERG, Nils-Olof, S-216 15 Malmö (SE)
(74) Representative: Hedenström, John Allan
(86) International application number: PCT/SE2002/001612
(87) International publication number: WO 2003/026656

(56) References cited:
- WO-A-03/026655
- WO-A1-00/30641
- GB-A- 2 147 501
- US-A- 5 662 920

## Description

### Field of the Invention

This invention relates to novel pharmaceutical compositions of nicotine and use thereof. More particularly, the present invention relates to compositions comprising nicotine and chocolate, methods to prepare said compositions, and to methods for using said compositions in nicotine replacement therapy (NRT), including tobacco substitution and smoking cessation

### Background and Prior Art

Nicotine replacement therapy as a smoking cessation strategy has been successful in the past. Previous nicotine-containing compositions aiming towards the purpose of reducing nicotine craving for subjects wishing to stop their use of tobacco products include e g US 3,845,217 disclosing chewable compositions, US 4,579,858 disclosing high-viscous nicotine nose-drop compositions, US 5,525,351 disclosing nicotine-containing saliva-soluble gels, US 5,656,255 disclosing low-viscous nicotine-containing compositions suitable for nasal spray administration, US 4,920,989 and US 4,953,572 disclosing the use of inhalation aerosol, BP 1,528,391 and BP 2,030,862 disclosing liquid aerosol formulations adapted as mouth-sprays, and devices for transdermal delivery of nicotine.

A well-known side effect of nicotine is related to its concentration dependent local irritation. This adverse effect is particularly noticeable when nicotine formulations are applied topically, including the transmucosal, comprising buccal and nasal, and transdermal administration routes.

UK Patent application GB 2 230 439 A describes nicotine lozenges with a shell or coating containing an oral-acting local analgesic, preferably eugenol. Though not stated explicitly to be the cause of the so included local analgesic, the aforesaid disclosure is said to substantially ameliorate the sensation of burning in the mouth experienced with conventional nicotine lozenges. Similarly, nicotine-compositions formulated in lozenges containing local analgesic have been disclosed in AU 662877 in which the latter agent is said to temporarily interfere with taste receptors which is said to reduce the desire to eat.

The concentration of nicotine in several of the above-mentioned inventions, and product designs thereof, is hence limited by adverse effects caused by or related to its local irritation.

Prior art describes other capsules, tablets, and lozenges for oral delivery of nicotine. For example, WO 88/03803 discloses a chewable capsule filled with a liquid containing 0.1-10.0 mg of nicotine, together with additives for improving flavour and dispersion. The capsules are provided in a variety of pH values to allow the patient a choice of nicotine absorption rates, and are especially intended as an aid to quit smoking.

Another nicotine capsule formulation is disclosed by Jarvik et al. (Clinical Pharmacology and Therapeutics 1970;11:574) for ingestion as a smoking cessation aid. The subjects, according to the theory that intestinal absorption of nicotine could produce significant blood levels, however, apparently swallowed these capsules whole. The study showed a small but significant decrease in the number of cigarettes smoked by subjects, but no quantitative measurements of nicotine blood levels were obtained.

BE 899037 discloses a tablet containing 0.1 to 5 mg nicotine as a base or watersoluble acid salt as an aid for quitting smoking.

Shaw (for example in GB 2 142 822 and US 4,806,356) describes a nicotine lozenge prepared from a mixture of inert filler material, a binder, and either pure nicotine or a nicotine-containing substance by cold compression.

US 5,512,306 discloses a nicotine product for oral delivery in the form of an inclusion complex of nicotine and a cyclodextrin compound. It also discusses the use of various excipients and direct compression for manufacture of the product.

US 5,662,920 discloses a nicotine lozenge that may contain candy taste flavorants, such as chocolate, orange, vanilla, as well as other flavorants. Their use also for taste-masking is though not suggested. Further, no amounts of these flavorants being sufficient for achieving a taste-masking effect is disclosed.

WO 97/42941 discloses a slowly erodible nicotine lozenge that allows delivery to the buccal mucosa over an extended period of time.

GB 2 147 501 A discloses an oral dosage form comprising a microencapsulated active principle embedded in a soft sweet palatable matrix. This matrix may be chocolate. Nicotine is not suggested as an active principle.

The literature describes different designs of tablets for delivering nicotine to the mouth and to the digestive system.

Wesnes and Warburton (Psychopharmacology 1984; 82:147; ibid. 1986;89:55) discuss the use of nicotine containing dextrose and magnesium hydroxide tablets. The subjects were instructed to keep the tablets in the mouth for some minutes before swallowing, in order to maximize contact with the buccal mucosa.

Several products based on the above mentioned patents are now marketed on an international scale. In addition, several nicotine lozenges are available as over-the-counter products in the UK Resolution lozenges, manufactured by Phoenix Pharmaceuticals and distributed by Ernest Jackson, contain 0.5 mg nicotine, together with the antioxidant vitamins A, C, and E. Stoppers lozenges, distributed by Charwell Pharmaceuticals Ltd., contain 0.5 mg nicotine and are available in chocolate, orange and peppermint flavours.

There are, however, subjects who may have cravings for higher doses of nicotine than those acceptable in applications of prior art and subjects that may not experience a decrease in other withdrawal symptoms because of unsatisfactory nicotine absorption. Furthermore, it has to date been difficult to deliver nicotine in a profile mimicking the nicotine blood levels achieved by consistent smoking, to satisfy cravings for nicotine in people who are attempting to quit smoking, and thus, to provide greater protection against relapse than nicotine replacement therapies is possible with hitherto known. Thus, absorption of nicotine in the use of currently marketed products and as disclosed in prior art of nicotine replacement therapies is not satisfactorily resembling the use of tobacco products, in particular smoking. With chewing gum nicotine replacement therapy for smoking cessation blood peak levels of nicotine is reached after 30 minutes with venous blood nicotine levels about 1/3 to 2/3 of the levels attained when smoking *(*British Medical Journal 1976;1:1043). A smoker will usually reach peak blood levels of nicotine 5 -10 minutes after starting smoking. It is therefore desirable to provide improved compositions which avoid the disadvantages of these conventional nicotine delivery devices while providing an effective means for delivering nicotine for smoking cessation treatment, for reducing nicotine craving, and for treating other conditions responsive to nicotine therapy.

An attempt to solve the captioned problems is made with a nicotine-containing composition, preferably for buccal uptake, according to WO 00/30641. Herein is disclosed a composition comprising nicotine, at least one apolar component, at least one polar component and at least one surface-active component. Many apolar components are suggested, including lipids such as cocoa butter and cocoa butter alternatives, including cocoa butter equivalents (CBE), cocoa butter substitutes (CBS), cocoa butter replacers (CBR) and cocoa butter improvers (CBI). Anyhow, the composition according to WO 00/30641 has the disadvantage of insufficient taste-masking of nicotine and buffering agents, and the drawback of causing nausea with some users. WO 00/30641 does not disclose chocolate as an ingredient.

It has now surprisingly been found that a rapid buccal absorption of nicotine concomitantly with sufficient taste-masking of badly tasting ingredients, such as buffering agents and nicotine, is achieved through the use of nicotine-containing formulations comprising chocolate as vehicle. No similar formulations have been disclosed hitherto.

Chocolate has hardly been used as a vehicle for human pharmaceutical products, although chocolate-like pharmaceutical products of types laxatives exist. Also exist chocolate-type veterinary products. Ex-Lax^{®}, being chocolated laxative pieces marketed by Novartis comprising sennosides, are formulated with a chocolate-like vehicle. In the 1950s was marketed Purex, a laxative wherein phenolphtaleine was formulated with chocolate. The Stoppers lozenges mentioned above do not comprise chocolate, but only chocolate flavours. Such chocolate flavours are not useful for the objectives of the present invention. It has not been disclosed use of chocolate as a vehicle for nicotine.

### Summary of the invention

Compositions for the therapeutic delivery of nicotine are provided. Said compositions comprising nicotine provide rapid transmucosal absorption of nicotine. The compositions are preferably used for therapeutic administration of nicotine.

The meaning of "disintegration" as used in the description and in the claims denotes melting, solubilization, erosion or a combinatorial effect of these physical changes of the invention.

In the absence of explicit statements to the contrary, as used herein expressions like "comprising", "including", "having", "with" and similar terminology shall not be understood to be exclusively restricted to the recited element(s), but shall be understood to allow for the presence of further elements as well, and shall be understood to cover any element(s) in integral, sub-divided or aggregate forms, as well to imply the inclusion of a stated integer or step or group of integers or steps, but not the exclusion of any other integer or step or group of integers or steps.

An object of the invention is to provide new pharmaceutical compositions of nicotine for buccal uptake, especially such compositions comprising a large percentage of chocolate. By "buccal" in the present application is meant "for uptake buccaly or by other mucosa in the oral cavity"

A second object of the invention is a use of said formulations in the manufacture of a medicament for nicotine replacement therapy (NRT), including tobacco substitution and smoking cessation.

### Detailed Description of the Invention

It is the primary object of the present invention to provide a tobacco supplement or a tobacco substitute, for use in e g smoking cessation and nicotine replacement therapies, which provide the user with a satisfactory dose of nicotine so as to reduce tobacco withdrawal symptoms without causing unacceptable adverse effects. More specifically it is the object of the invention to provide such a nicotine containing tablet, for transmucosal, preferably buccal, delivery, which disintegrates and/or melts at body temperature with or without the aid of salivary fluid or mechanical erosion, or a combination thereof after which the formulation preferably shows adhesiveness towards the tissues in the oral cavity.

The nicotine may be present in any suitable form, eg as free base, as a salt or as a complex. There is no need to use nicotine in a microencapsulated form.

The formulation is a tablet melting in the mouth, weighing 400 mg, having the following preferred composition.
Nicotine (as base or hydrogen tartrate) 1 - 6 mg measured as base,
Sodium carbonate 15 mg,
Dark chocolate 95% (w/w)

According to Industrial Chocolate Manufacture and Use, S. T. Beckett, ed., 2nd edition, Blackier Academic & Professional, London, 1994, p.382, chocolate is defined as a product obtained from cocoa nib, cocoa mass powder and sucrose with or without added cocoa butter, having a minimum dry cocoa solids content of 35%, at least 14% of dry non-fat cocoa solids and 18% cocoa butter. Chocolate has two major distinguishing characteristics: its flavor and its texture. A primary feature of the texture is that the chocolate must be solid at a temperature of 20 - 25°C and yet melt rapidly in the mouth at 37°C thereby being transferred to a liquid, which appears smooth to the tongue. The processing of chocolate is related to obtaining these two criteria (*ibid.* p 2). The higher the content of dry cocoa solids in the chocolate the better the taste masking effect of the chocolate in the present invention. Chocolate may also be defined according to different national directives, such as European Council Directive 2000/36/EC of 23 June 2000, the old Council Directive 73/241/EEC of 24 July 1973 (to be repealed from 3 August 2003) and the US directive 21 CFR CH 1 (edition 4-1-00), part 163 Cacao products.

### Example 1: Preparation

A tablet, weighing 400 mg, having the following preferred composition (w/w):

| | |
|---|---|
| Active: | nicotine (as base or salt, preferably hydrogen tartrate) 1 - 6 mg measured as base, |
| | The nicotine may also be present in a complex, e g with a cation exchange resin or with cyclodextrin. |
| Buffering agent: | sodium carbonate 15 mg |
| Vehicle: | dark chocolate 95% (w/w) |

is prepared in the following way:

A part of the chocolate is melted. The solid components, i e nicotine, if in salt form, and sodium carbonate are added and mixed. A reduction of particle size of the solid components is performed by milling in a roll-refiner. If the solid components have already got the required particle size, e g by milling before the mixing with the chocolate, roll refining is dispensed with. After treatment in the roll-refiner the mixture is mixed with the rest of the melted chocolate or remelted (if solidified) and mixed with the rest of the melted chocolate. Chocolate can be used as a raw material. Chocolate can also be produced in connection with the production of the embodiment. A mixing of the melt is performed in a suitable mixer. The liquid component, i e nicotine, if in liquid base form, is added. When chocolate is used as raw material a certain percentage of lecithin is already included (normally around 0.3%). Tablets or other solid dosage forms are subsequently made using suitable techniques, such as molding, extrusion or congealing, including pastillation, if necessary after suitable preconditioning. Also other suitable manufacturing methods may be used.

Optionally flavoring agents, such as mint, coffee, orange, vanilla and milk-butterscotch, may be added.

The present nicotine-containing composition may be administered in combination with a second formulation for nicotine replacement therapy. This second formulation may be a device for transdermal administration of nicotine, a spray for nasal, buccal or pulmonary uptake, a chewing gum, or a dosage form for oral or peroral use or any device for administration of tobacco.

The present invention may also be used in cessation, reduction and temporary abstinence of tobacco, and for treatment of Alzheimer's disease, Parkinson's disease, ulcerative colitis and/or Tourette's syndrome; and/or weight control therapy.

## Claims

1. A nicotine-containing pharmaceutical composition, which is formulated as an oral dosage form and which is suitable for delivery of nicotine through the buccal mucosa, **characterized in that** a unit dose thereof, weighing 400 mg, comprises
| | |
|---|---|
| Nicotine: | from 1 mg to 6 mg, measured as base, |
| Buffering agent(s): | 15 mg sodium carbonate, |
| Dark chocolate vehicle: | 95% (w/w). |

2. A nicotine-containing pharmaceutical composition according to claim 1, **characterized in that** it further comprises one or more flavoring agents, such as mint, coffee, orange, vanilla and milk-butterscotch.

3. Use of a nicotine-containing pharmaceutical composition according to anyone of the preceding claims for the manufacture of a medicament for nicotine replacement therapy (NRT), cessation, reduction and temporary abstinence of tobacco, and for treatment of Alzheimer's disease, Parkinson's disease, ulcerative colitis and/or Tourette's syndrome; and/or weight control therapy.

## Patentansprüche

1. Nikotinhaltige pharmazeutische Zusammensetzung, die als orale Darreichungsform formuliert ist und geeignet ist zum Zuführen von Nikotin durch die Mundschleimhaut, **dadurch gekennzeichnet, dass** eine Einzeldosis davon mit einem Gewicht von 400 mg umfasst:
Nikotin: von 1 mg bis 6 mg, gemessen als Base,
Puffermittel: 15 mg Natriumcarbonat,
Bitterschokolade-Vehikel: 95% (w/w).

2. Nikotinhaltige pharmazeutische Zusammensetzung nach Anspruch 1, **dadurch gekennzeichnet, dass** diese ferner ein oder mehrere Geschmacksstoffe, wie Minze, Kaffee, Orange, Vanille und Milch-Karamell, umfasst.

3. Verwendung einer nikotinhaltigen pharmazeutischen Zusammensetzung nach einem der vorhergehenden Ansprüchen zur Herstellung eines Medikaments für die Nikotinersatztherapie (NRT), Tabakentwöhnung, Tabakeinschränkung und zeitweilige Tabakabstinenz, und zur Behandlung von Alzheimer-Krankheit, Parkinson-Krankheit, Colitis ulcerosa und/oder Tourette-Syndrom; und/oder zur Gewichtskontrolltherapie.

## Revendications

1. Composition pharmaceutique contenant de la nicotine, qui est formulée en une forme galénique orale et qui est appropriée à la délivrance de nicotine à travers la muqueuse buccale, **caractérisée en ce qu'**une dose unitaire, pesant 400 mg, comprend
| | |
|---|---|
| nicotine: | de 1 mg à 6 mg, mesurée en tant que base, |
| agents(s) de tamponnage: | 15 mg de carbonate de sodium, |
| véhicule chocolat noir: | 95 % en masse. |

2. Composition pharmaceutique contenant de la nicotine selon la revendication 1, **caractérisée en ce qu'**elle contient en outre un ou plusieurs agents aromatisants, comme de la menthe, du café, de l'orange, de la vanille et du caramel au lait.

3. Utilisation d'une composition pharmaceutique contenant de la nicotine selon l'une quelconque des revendications précédentes pour la fabrication d'un médicament destiné à une thérapie de remplacement de la nicotine, à la cessation, la réduction et l'abstinence temporaire du tabac, et au traitement de la maladie d'Alzheimer, de la maladie de Parkinson, de la colite ulcéreuse et/ou du syndrome de Tourette; et/ou à une thérapie de perte de poids.
